# EUROPÄISCHE PATENTSCHRIFT

(11) **EP 0 641 596 B1**
(45) Veröffentlichungstag und Bekanntmachung des Hinweises auf die Patenterteilung: **16.12.1998**
(21) Anmeldenummer: 94113270.6
(22) Anmeldetag: 25.08.1994
(51) Int. Cl.: B01J 2/04, B01J 2/06, B01J 13/04, C09B 61/00, C09B 67/00

(54) **Verfahren zur Herstellung von feinteiligen Farb- oder Wirkstoffzubereitungen**
Process for preparing finely divided colouring or active agents
Procédé pour la préparation de colorants ou d'agents actifs finement divisés

(30) Priorität: 01.09.1993 DE 4329446
(43) Veröffentlichungstag der Anmeldung: 08.03.1995
(73) Patentinhaber: BASF Aktiengesellschaft, 67063 Ludwigshafen (DE)
(72) Erfinder: End, Dr. Lutz, D-68199 Mannheim (DE); Horn, Dr. Dieter, D-69120 Heidelberg (DE); Lueddecke, Dr. Erik, D-67112 Mutterstadt (DE)

(56) Entgegenhaltungen:
- EP-A- 0 155 239
- EP-A- 0 275 796
- EP-A- 0 278 284
- EP-A- 0 410 236

## Beschreibung

Die Erfindung betrifft ein verbessertes Verfahren zum Mikronisieren von in Wasser schwer löslichen Verbindungen.

Es existieren verschiedene Verfahren zur Herstellung kleiner Teilchen von schwer in Wasser löslichen Verbindungen, wie sie zur Erhöhung der Bioverfügbarkeit von schwer löslichen Wirkstoffen oder der Farbstarke von schwer löslichen Farbstoffen benötigt werden. So beschreiben beispielsweise EP 65 193, EP 169 618 und US 5 133 908 Verfahren, bei denen der Wirk- oder Farbstoff in eine molekulare Lösung in eine wassermischbaren Lösungsmittel überführt und anschließend durch Zugabe der Wirkstofflösung zu Wasser oder umgekehrt in feinteiliger Form wieder ausgefällt wird. Diese Verfahren weisen jedoch den Nachteil auf, daß zur Formulierung des Wirkstoffs in feinteiliger Form Lösungsmittel in größerer Menge verwendet werden müssen, um den (oft auch in organischen Lösungsmitteln schwer löslichen) Wirk- oder Farbstoff zu lösen.

EP 410 236 beschreibt ein Verfahren zur Herstellung von Carotinoidpräparaten, bei denen man eine Suspension des Carotinoids in einem hochsiedenden Öl kurzzeitig mit überhitztem Wasserdampf in Kontakt bringt, anschließend emulgiert und die Emulsion sprühtrocknet. Nachteilig an diesem Verfahren ist, daß das erhaltene Trockenpulver, aufgrund des hohen Ölanteils, nur eine geringe Wirkstoffkonzentration aufweist.

Ziel der vorliegenden Erfindung war es daher, die Menge des verwendeten Lösungsmittels möglichst zu verringern und dennoch zu äußerst feinteiligen Formulierungen der Farb- und Wirkstoffe zu gelangen.

Diese Verringerung der Menge des benötigten Lösungsmittels gelingt bei der erfindungsgemäßen Verfahrensweise dadurch, daß keine zwischenzeitliche molekulare Lösung des Wirk- oder Farbstoffes in einem wassermischbaren Lösungsmittel hergestellt werden muß, sondern das Verfahren so gefuhrt wird, daß der in wäßriger oder organischer Phase dispergierte Wirk- oder Farbstoff kurzzeitig unter heftiger Turbulenz einer Temperatur über seinem Schmelzpunkt ausgesetzt und die so erhaltene feinteilige Emulsion anschließend durch schnelles Abkühlen unter den Schmelzpunkt in eine stabile Dispersion des Feststoffs in Wasser überführt wird. Diese kann anschließend in bekannter Weise aufkonzentriert und getrocknet werden, oder die Emulsion kann sofort, ohne zwischenzeitliche Abkühlung, sprühgetrocknet werden. In beiden Fällen erhält man so Pulver, die bei der Redispergierung in Wasser eine feinteilige Dispersion des Farb- oder Wirkstoffes ergeben.

Die Vermeidung oder Verringerung des Lösungsmittelbedarfs während der Formulierung hat nicht nur den Vorteil geringerer Kosten, sondern sie ermöglicht z.B. im Falle des Fenofibrates erst die Herstellung von stabilen Nanopartikeln, weil bei der Herstellung über wäßrige Dispersionen von festen Partikeln diese sofort über Ostwaldt-Reifung zu größeren Partikeln wachsen und so der Mikronisiererfolg zunichte gemacht wird. Die Ostwaldt-Reifung wird bei den hier intermediär kurzzeitig hergestellten Emulsionen nicht beobachtet. Sie wird zusätzlich durch das vollständige oder weitgehende Vermeiden eines Lösungsmittels (und die dadurch bedingte Verminderung der Restlöslichkeit im Dispergiermedium) soweit verzögert, daß während der sofortigen Sprühtrocknung kein Partikelwachstum beobachtet werden kann.

Es gibt allerdings Stoffe, die einer ganz lösungsmittelfreien Verarbeitung nicht zugänglich sind, weil ihre wäßrigen Dispersionen klumpen und daher nicht dosierbar sind. Ein Beispiel dafür ist Fenofibrat. Solche Stoffe werden daher erfindungsgemäß, statt in Wasser dispergiert, in möglichst wenig organischem Lösungsmittel gelöst (falls sie in dem betreffenden Lösungsmittel leicht löslich sind) oder dispergiert. Eine vollständige Lösung ist nicht erforderlich, es genügt, wenn die Suspension dosierbar ist. Zu dieser Lösung oder Suspension gibt man dann unter heftiger Turbulenz, und gegebenenfalls unter Druck, Wasser oder eine wäßrige Schutzkolloidlösung von einer Temperatur oberhalb des Schmelzpunktes des jeweiligen Stoffes und verfährt im übrigen wie vorher beschrieben, also sofortiges und unmittelbares Sprühtrocknen der Schmelzemulsion oder rasches Abkühlen der entstandenen feinteiligen Emulsion und gegebenenfalls Entwässern und Trocknen der so erhaltenen Suspension. Das rasche Abkühlen kann zweckmäßig durch Zusatz kalten Wassers erreicht werden.

Wie die Turbulenz beim Zumischen des Wassers oder der wäßrigen Schutzkolloidlösung zur Suspension oder Lösung des Farb- oder Wirkstoffs erzeugt wird, ist nebensächlich. Es kann z.B. heftig gerührt oder geschüttelt werden. Am einfachsten und daher bevorzugt ist das Einspritzen einer oder besser beider Komponenten in hartem Strahl, so daß es ohne mechanische Hilfsmittel sofort zu einer innigen Durchmischung kommt.

Mit "feinteilig" oder "kolloidaler Verteilung" sind Teilchengrößen unter 1,5, vorzugsweise unter 1 µm gemeint.

"Im wesentlichen amorph" heißt, daß mehr als die Hälfte, vorzugsweise mehr als 70, insbesondere annähernd 100 % des erfindungsgemäß hergestellten Produktes röntgenamorph sind.

Mit Wirkstoffen sind pharmazeutische Wirkstoffe gemeint. Für sie ist die Erfindung noch wichtiger als für Farbstoffe, denn bei Farbstoffen bringt die Erfindung "nur" den Vorteil, daß aufgrund der feineren Verteilung der gleiche Färbungseffekt mit weniger Farbstoff erzielt wird, während es bei Wirkstoffen vor allem um die Verbesserung der Resorbierbarkeit schwer oder unlöslicher Wirkstoffe geht, eine für die Wirkung entscheidende Größe.

Der in Betracht kommende Temperaturbereich beim Mischen umfaßt 40 bis 250, vorzugsweise 60 bis 200°C.

Schutzkolloide dienen der Stabilisierung der zunächst entstehenden Emulsion und der daraus beim Abkühlen entstehenden Dispersion. Außerdem gewährleisten sie die leichte Redispergierbarkeit des gegebenenfalls erfindungsgemäß hergestellten Pulvers. Schutzkolloide sind z.B. Gelatinen verschiedener Herkunft, Casein, Gummi arabicum, Lysalbinsäure, Stärke, Dextrin, Pektin, Methylcellulose, Carboxymethylcellulose, Hydroxypropylcellulose, Alginate, Polyvinylalkohol, Polyvinylpyrrolidon, Polyacrylate.

Mit "relativ grobteilig" ist gemeint, grobteilig im Verhältnis zum Endprodukt. Die Teilchengröße ist praktisch unerheblich und kann im Bereich von etwa 1 bis 2.000 µm liegen.

Als Lösungsmittel kommt im Prinzip jedes in Betracht, das zu mindestens 10 % wasserlöslich ist. Außerdem muß es unzersetzt destillierbar sein. Als Beispiele seien genannt: Alkohole, Äther, Ketone, Ester, Acetale, Dimethylformamid, N-Methylpyrrolidon, Dimethylsulfoxid.

Zur Erhöhung der mechanischen Stabilität des Endproduktes ist es zweckmäßig, dem Kolloid einen Weichmacher oder Füllstoff zuzusetzen, wie Zucker oder Zuckeralkohole, z.B. Saccharose, Glukose, Lactose, Invertzucker, Sorbit, Mannit oder Glycerin.

Das Endprodukt enthält, wenn es als trockenes Pulver vorliegt, 0,5 bis 20, vorzugsweise etwa 10 Gew.-% des schwerlöslichen Farb- oder Wirkstoffs, 10 bis 50 Gew.-% Schutzkolloid und 30 bis 80 Gew.-% eines Weichmachers sowie ggf. geringe Mengen weiterer Hilfsmittel wie Emulgatoren, Antioxidantien und andere Stabilisatoren.

Im Fall der Verwendung eines organischen Lösungsmittels kann die Entfernung des Lösungsmittels je nach Siedepunkt in an sich bekannter Weise, z.B. durch Destillation, gegebenenfalls unter vermindertem Druck, oder durch Extraktion mit einem mit Wasser nicht mischbaren Lösungsmittel, erfolgen. In diesem Falle hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt sie jedoch gemeinsam mit der Entfernung des Wassers durch Sprühtrocknung oder Sprühgranulation.

Man erhält ein Trockenpulver, das bei Verwendung eines wasserlöslichen Schutzkolloids erneut in Wasser unter Erzielung einer gleichmäßigen Feinverteilung des Wirkstoffes im Korngrößenbereich < 1 µm gelöst werden kann. Im photochemischen Stabilitätstest erweist sich das so erhaltene Wirkstoff-Hydrosol trotz der Feinverteilung als außerordentlich stabil.

Geeignete Farb- und Wirkstoffe sind beispielsweise Carotinoide, Verapamil, Anipamil, Propaphenon, Biperiden.

Die in den Beispielen genannten Teile beziehen sich auf das Gewicht.

### Beispiel 1

### Lösungsmittelfreie Mikronisierung von β-Carotin

### Farbstoffdispersion:

21 Teile β-Carotin und 2,3 Teile Ascorbylpalmitat wurden in 240 Teilen Wasser mittels eines Magnetrührers dispergiert.

### Schutzkolloidlösung:

57,9 Teile Gelatine Typ B, 100 Bloom, und 97,5 Teile Lactose wurden in 4000 Teilen Wasser bei 70°C gelöst. Die so erhaltene Lösung wurde auf 25°C abgekühlt.

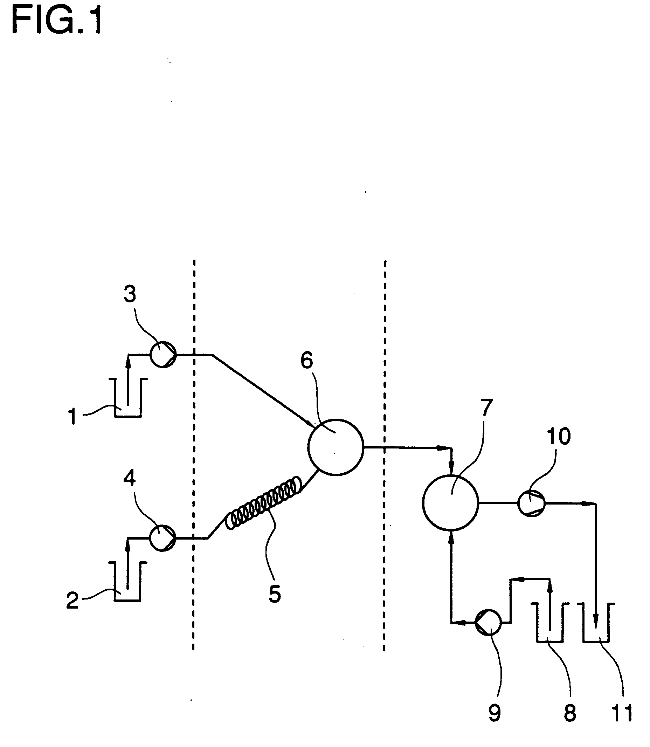

Die Herstellung des Mikronisates ist in Fig. 1 schematisch dargestellt. Die Farbstoffdispersion (1) wurde von der Pumpe (3) mit einer Pumprate von 230 Teilen/Stunde in die erste Mischzelle (6) gepumpt, in der sie mit einem Wasserstrom von 4740 Teilen/Stunde aus Gefäß (2) über die Pumpe (4) gemischt wurde, der vorher über den Thermostat (5) auf 227°C erhitzt worden war. Durch die Vermischung mit dem heißen Wasser wurde in der Mischung schlagartig eine Temperatur von 217°C erreicht und der Schmelzpunkt des β-Carotins (184°C) überschritten. Nach einer Verweilzeit von 0,5 s bei 217°C wurde die so erhaltene Emulsion des β-Carotins in der zweiten Mischzelle (7) mit der Schutzkolloidlösung (8) vermischt, die von der Pumpe (9) mit einer Flußrate von 32.200 Teilen/Stunde gepumpt wurde. Das so erhaltene feinteilige Mikronisat wurde über das Druckbegrenzerventil (10) ausgetragen. Die so hergestellten β-Carotinpartikel hatten eine mittlere Teilchengröße von 576 nm (bestimmt über Photonenkorrelationsspektroskopie) und lagen in einer kolloidal stabilen Dispersion vor.

### Beispiel 2

### Herstellung eines Mikronisates von Fenofibrat aus einer Emulsion mit 30 % Feststoffgehalt

### Wirkstofflösung:

500 Teile Fenofibrat wurden bei 50°C in 1500 Teilen Isopropanol gelöst.

### Schutzkolloidlösung:

125 g Ascorbylpalmitat wurden bei pH 9 (Zugabe von Natronlauge 1 M) unter Rühren in 10.000 Teilen Wasser von 70°C gelöst. In der so erhaltenen Lösung wurden 2000 Teile Gelatine Typ B, 30 Bloom, und 2875 Teilen Lactose unter Rühren aufgelöst.

### Durchführung der Mikronisierung:

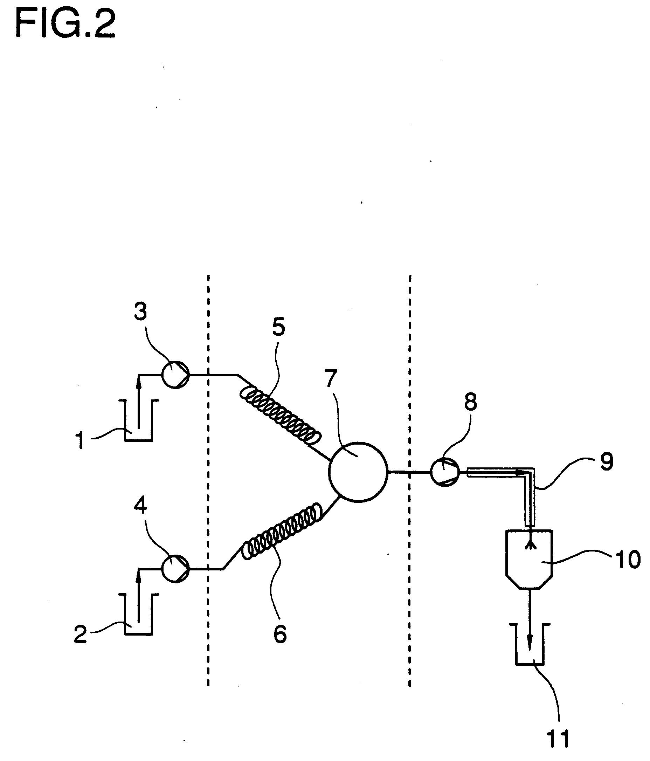

Die Herstellung des Mikronisates ist in Fig. 2 schematisch dargestellt. Die Wirkstofflösung (1) wurde bei 50°C, die Schutzkolloidlösung (2) bei 80°C vorgehalten. Die Wirkstofflösung wurde mit 2000 Teilen/Stunde von der Pumpe (3) über den Wärmetauscher in einem Ölbad von 120°C (5) in die Mischzelle (7) gepumpt. In der Mischzelle wurde die Wirkstofflösung mit der Schutzkolloidlösung (2) gemischt, die von der Pumpe (4) mit einer Flußrate von 9000 Teilen/Stunde gepumpt wurde und einen auf 130°C geheizten Wärmetauscher (6) passierte. Es stellte sich eine Mischtemperatur von 92°C ein, die über dem Schmelzpunkt des Fenofibrates von 80°C liegt. Die so erhaltene Emulsion wurde über das Druckbegrenzungsventil (8) ausgetragen, über den beheizten Schlauch (9) direkt in den Sprühturm (10) gefördert und in diesem bei einer Zulufttemperatur von 160°C und einer Ausgangstemperatur von 80°C getrocknet. Man erhielt auf diese Weise ein frei fließendes Pulver (11), das bei der Dispergierung in Wasser eine kolloidale Dispersion von Fenofibrat mit einer Partikelgröße von 0,66 µm (Volumenmittel, bestimmt über Laserbeugung) ergab.

### Beispiel 3

### Herstellung eines Mikronisates von Levemopamilhydrochlorid

### Wirkstoffdispersion:

9 g Levemopamil · HCl wurden mit 1,8 g Ascorbylpalmitat in einer Mischung aus 31,7 g Isopropanol und 4,3 g Wasser dispergiert.

### Schutzkolloidlösung:

Eine Lösung von 15 g Gelatine Typ B, 100 Bloom und 22,5 g Lactose in einem Liter Wasser wurde mit NaOH auf den pH-Wert 11,4 gebracht.

### Durchführung der Mikronisierung:

Die Herstellung des Mikronisates ist in Fig. 1 schematisch dargestellt. Die Wirkstoffdispersion (1) wurde mit 0,55 kg/h von der Pumpe (3) in die erste Mischzelle (6) gepumpt, in der sie mit einem Wasserstrom von 1 kg/h aus Gefäß (2) über die Pumpe (4) gemischt wurde, der vorher über den Thermostat (5) auf 210°C erhitzt wurde. Durch die Vermischung mit dem heißen Wasser stellte sich schlagartig eine Temperatur von 185°C ein, und der Schmelzpunkt des Levemopamil · HCl (182°C) wurde überschritten. Nach einer Verweilzeit von weniger als 0,5 s bei 185°C wurde die so erhaltene Emulsion von Levemopamil · HCl in der zweiten Mischzelle (7) mit der Schutzkolloidlösung (8) vermischt, die über die Pumpe (9) mit einer Flußrate von 9 kg/h gepumpt wurde. Das erhaltene Mikronisat wurde über das Druckbegrenzerventil (10) ausgetragen. Sprühtrocknung des Mikronisates ergab ein Pulver mit einem Wirkstoffgehalt von 22,3 %. Nach Auflösen des Pulvers in Wasser erhielt man eine mittlere Teilchengröße der Levemopamil · HCl-Nanopartikel von 580 nm (gemessen über Dynamische Lichtstreuung).

### Beispiel 4

### Herstellung eines Mikronisates von Propafenon · HCl.

### Wirkstoffdispersion:

Entsprechend Levemopamil · HCl (Beispiel 3), jedoch mit Propafenon · HCl.

### Schutzkolloidlösung:

Entsprechend Beispiel 3, jedoch mit pH-Wert 11,0.

### Herstellung des Mikronisates:

Die Herstellung des Mikronisates entsprach der Mikronisierung wie unter Beispiel 3 beschrieben. Die Mischtemperatur in der Mischzelle (6) war 177°C und damit über dem Schmelzpunkt des Propafenon · HCl (174°C). Durch Sprühtrocknung wurde ein Pulver mit 23,3 % Wirkstoff erhalten, die Teilchengröße der Nanopartikel nach Wiederauflösung des Pulvers in Wasser lag bei 350 nm (Dynamische Lichtstreuung).

### Beispiel 5

### Herstellung einer Dispersion von Nanopartikeln von Anipamil · HCl

### Herstellung der Wirkstoffdispersion:

21 g Anipamil · HCl wurden mit 0,24 g 10fach oxethylierten Isononylphenols in 240 g Wasser dispergiert.

### Herstellung der Schutzkolloidlösung:

Eine Lösung von 15 g/l Gelatine Typ B, 100 Bloom wurde mit NaOH auf pH 9 gebracht.

### Durchführung der Mikronisierung:

Die Durchführung der Mikronisierung entsprach der unter Beispiel 4 mit folgenden Änderungen:

| | |
|---|---|
| Pumprate Dispersion: | 2 kg/h |
| Pumprate Wasser: | 4 kg/h |
| Pumprate Schutzkolloidlösung: | 30 kg/h |
| Temperatur Wärmetauscher: | 100°C |
| Temperatur 1. Mischzelle (6): | 93°C |
| Schmelzpunkt Anipamil · HCl: | 63°C. |

Die so erhaltene Dispersion von Nanopartikeln des Anipamil · HCl in Wasser wies eine mittlere Teilchengröße von 220 nm (Dynamische Lichtstreuung) auf. Der Wirkstoffgehalt in dieser nanopartikulären Dispersion betrug 0,45 %.

### Beispiel 6

### Mikronisierung von Canthaxanthin

### Herstellung der Wirkstoffdispersion:

21 g Canthaxanthin wurden mit 4,6 g Ascorbylpalmitat in einer Mischung von 223 g Wasser und 12 g 1 M NaOH dispergiert.

Herstellung der Schutzkolloidlösung entsprechend Beispiel 5.

Die Durchführung der Mikronisierung entsprach der unter Beispiel 4 mit folgenden Änderungen:

| | |
|---|---|
| Pumprate Dispersion: | 1,1 kg/h |
| Pumprate Wasser: | 6 kg/h |
| Pumprate Schutzkolloidlösung: | 30 kg/h |
| Temperatur Wärmetauscher: | 240°C |
| Temperatur 1. Mischzelle (6): | 223°C |
| Schmelzpunkt Canthaxanthin | 211°C. |

Die so erhaltene Dispersion von Nanopartikeln des Canthaxanthins in Wasser wies eine mittlere Teilchengröße von 370 nm (Dynamische Lichtstreuung) auf. Der Wirkstoffgehalt in der nanopartikulären Dispersion lag bei 0,21 %.

### Beispiel 7

### Herstellung eines Mikronisates von Nesapidil

### Herstellung der Wirkstoffdispersion:

3 g Nesapidil wurden mit 0,6 g Ascorbylpalmitat in einer Mischung aus 31,7 g Isopropanol und 4,3 g Wasser dispergiert.

Herstellung der Schutzkolloidlösung entsprechend Beispiel 5.

### Durchführung der Mikronisierung:

Die Durchführung der Mikronisierung entsprach der unter Beispiel 4 mit folgenden Änderungen:

| | |
|---|---|
| Temperatur Wärmetauscher: | 210°C |
| Temperatur 1. Mischzelle (6): | 175°C |
| Schmelzpunkt Nesapidil: | 164°C. |

Durch Sprühtrocknung wurde ein Pulver mit einem Nesapidilgehalt von 10,3 % erhalten. Die mittlere Teilchengröße nach Wiederauflösung des Pulvers in Wasser lag bei 350 nm (Dynamische Lichtstreuung).

## Patentansprüche

1. Verfahren zur Herstellung von feinteiligen, im wesentlichen amorphen Farb- oder Wirkstoffzubereitungen durch Überführen einer relativ grobteiligen Dispersion mit einer Teilchengröße von etwa 1 bis 2000 µm oder einer Lösung des Farb- oder Wirkstoffs in einem organischen Lösungsmittel, das zu mindestens 10 % wasserlöslich ist, in einen kolloidalen Verteilungszustand in Wasser, dadurch gekennzeichnet, daß die wäßrige Dispersion oder organische Lösung des Farb- oder Wirkstoffs bei einer Temperatur oberhalb des Schmelzpunktes des Farb- oder Wirkstoffs (gegebenenfalls unter Druck) mit Wasser oder einer wäßrigen Schutzkolloidlösung turbulent vermischt und die so erhaltene Schmelzemulsion sofort sprühgetrocknet oder durch Abkühlen in eine Suspension umgewandelt wird.

2. Verfahren nach Anspruch 1, dadurch gekennzeichnet, daß man ohne organische Lösungsmittel arbeitet.

3. Verfahren nach den Ansprüchen 1 und 2, dadurch gekennzeichnet, daß das Abkühlen möglichst rasch nach der Bildung der heißen Schmelz-Emulsion erfolgt.

4. Verfahren nach einem der Ansprüche 1 bis 3, dadurch gekennzeichnet, daß man die erhaltene Suspension durch Sprühtrocknung oder Sprühgranulation vom Wasser oder Wasser/Lösungsmittelgemisch befreit und so ein leicht redispergierbares Pulver gewinnt.

## Claims

1. A process for producing fine-particle, essentially amorphous dye or drug preparations by converting a relatively coarse-particle dispersion having a particle size of about 1 to 2000 µm or a solution of the dye or drug in an organic solvent which is at least 10% water-soluble into a colloidal dispersion in water, wherein the aqueous dispersion or organic solution of the dye or drug is subjected to turbulent mixing at a temperature above the melting point of the dye or drug (where appropriate under pressure) with water or an aqueous protective colloid solution, and the resulting melt emulsion is immediately spray-dried or converted into a suspension by cooling.

2. A process as claimed in claim 1, wherein no organic solvent is used.

3. A process as claimed in claim 1 or 2, wherein the cooling takes place as quickly as possible after the formation of the hot melt emulsion.

4. A process as claimed in any of claims 1 to 3, wherein water or water/solvent mixture is removed from the resulting suspension by spray drying or spray granulation to afford an easily redispersable powder.

## Revendications

1. Procédé pour la préparation de compositions de colorants ou de substances actives essentiellement amorphes en fines particules par conversion d'une dispersion à particules relativement grossières, à une dimension de particule d'environ 1 à 2000 µm ou d'une solution du colorant ou de la substance active dans un solvant organique soluble à au moins 10 % dans l'eau, en un état de dispersion colloïdale dans l'eau, caractérisé par le fait que l'on soumet la dispersion aqueuse ou solution organique du colorant ou de la substance active à mélange turbulent à une température supérieure au point de fusion du colorant ou de la substance active (éventuellement sous pression) avec de l'eau ou une solution aqueuse d'un colloïde protecteur, ce qui donne une émulsion de matière fondue qu'on soumet immédiatement à séchage par atomisation ou qu'on convertit en une suspension par refroidissement.

2. Procédé selon la revendication 1, caractérisé par le fait que l'on opère en l'absence de solvant organique.

3. Procédé selon les revendications 1 et 2, caractérisé par le fait que le refroidissement est réalisé aussi rapidement que possible après la formation de l'émulsion chaude de matière fondue.

4. Procédé selon une des revendications 1 à 3, caractérisé par le fait que l'on débarrasse la suspension obtenue de l'eau ou du mélange eau/solvant par séchage par atomisation ou granulation par pulvérisation, ce qui donne une poudre bien redispersable.
